# FASCICULE DE BREVET EUROPEEN

(11) **EP 1 695 663 B1**
(45) Date de publication et mention de la délivrance du brevet: **07.12.2016**
(21) Numéro de dépôt: 06300162.2
(22) Date de dépôt: 24.02.2006
(51) Int. Cl.: A61B 5/103, A61B 10/00, G01N 33/50

(54) **Procédé pour évaluer le potentiel de désactivation de radicaux libres de la peau**
Verfahren zur Auswertung des Potentials der Haut zur Deaktivierung von freien Radikalen
Method for evaluating the skin potential of deactivating free radicals

(30) Priorité: 25.02.2005 FR 0550525
(43) Date de publication de la demande: 30.08.2006
(73) Titulaire: L'Oréal, 75008 Paris (FR)
(72) Inventeur: Pruche, Francis, 60300 Senlis (FR); Nguyen, Quang Lan, 92160 Antony (FR); Hirt, Jean-Pascal, 92210 Saint-Cloud (FR)
(74) Mandataire: Nony

(56) Documents cités:
- EP-A- 1 262 559
- FR-A1- 2 844 986
- US-A- 4 595 011
- US-A- 5 667 501
- US-A- 6 093 409
- US-A- 6 108 570
- US-B1- 6 316 012
- FUCHS J. ET AL.: "HPLC analysis of vitamin E isoforms in human epidermis: correlation with minimal erythema dose and free radical scavenging activity." FREE RADIC. BIOL. MED., vol. 34, no. 3, 1 février 2003 (2003-02-01), pages 330-336, XP002348232
- THIELE J.J. ET AL.: "Sebaceous gland secretion is a major physiologic route of vitamin E delivery to skin." J. INVEST. DERMATOL., vol. 113, no. 6, 1999, pages 1006-1010, XP002348233
- MONDON P. ET AL.: "Evaluation of free-radical scavenger effects of Helianthus annuus extracts using new ex vivo stripping methods", COSMETICS, AEROSOLS & TOILETRIES IN AUSTRALIA, vol. 12, no. 4, 1999, pages 20-26, XP009178845,

## Description

La présente invention concerne les procédés et dispositifs permettant d'évaluer le potentiel de désactivation de radicaux libres de la peau, y compris les muqueuses, ce potentiel étant encore appelé potentiel antioxydant.

Les radicaux libres nocifs sont des molécules à durée de demi-vie très courte, de l'ordre de la nanoseconde à la milliseconde, donc très instables et très réactifs.

On sait que la capacité de la peau à se protéger d'agressions extérieures, telles que l'exposition aux rayons ultraviolets, la pollution ou autres agressions biologiques ou chimiques, peut dépendre notamment de sa teneur épidermique en substances antioxydantes non enzymatiques capables de désactiver les radicaux libres.

Ainsi, la vitamine E est une substance antioxydante connue pour protéger la peau et prévenir son vieillissement, que l'on retrouve fréquemment dans les formulations cosmétiques.

Selon les individus, la concentration dans la peau en substances antioxydantes telles que les molécules à fonction SH, l'ascorbate, le tocophérol, le tocotriénol, l'acide urique, la vitamine A, l'ubiquinone 10, la spermine, ... peut varier, par exemple en raison de l'âge, de la nature de la peau, de l'alimentation, du degré d'exposition aux ultraviolets, etc ...

Le suivi direct des radicaux libres est inabordable dans les conditions courantes d'appareillage.

Il a été proposé d'utiliser des molécules neutres capteurs appelées *spin trap,* qui donnent par réaction des molécules radicalaires plus stables et ainsi mesurables par *ESR (Election Spin Resonance).*

La formation de peroxydes lipidiques ou de peroxydes de squalène comme indicateur du stress cutané a été étudiée dans la publication Ultraviolet A induces generation of squalene monohydroperoxide isomers in human sebum and skin surface lipids in vitro and in vivo: Mudiyanselage SE, Hamburger M, Elsner P, Thiele JJ/J Invest Dermatol. 2003 June; 120(6) 915-22 et dans la demande de brevet allemand DE 10 164 553.

L'article Evaluation of free radical scavanger effects of Helianthus annus extract using new ex vivo stripping methods: P. Mondon et al. dans Cosmetics, aerosols & toiletries in Australia 20/05/1999 décrit une méthode consistant à prélever un échantillon de *stratum corneum* et à en extraire les substances antioxydante, que l'on fait réagir avec un réactif coloré qui est le DPPH (2,2-diphényl-1-picrymydrazyl), la décoloration de ce réactif étant suivie avec un spectrophotomètre à 517 nm.

Pour sa part, le document J. Fuchs et al., Radical Biology and Medecine, Vol. 34, No.3. pp; 330-336, décrit la mise en oeuvre de ce réactif dans la méthode de résonance paramagnétique électronique (Electron Paramagnetic Resonance, en anglais) pour analyser le contenu en substances antioxydantes à l'image d'isoformes de la vitamine E dans un échantillon de peau, obtenu par biopsie.

Le DPPH est un radical libre stable qui, en présence d'un ou plusieurs désactivateurs de radicaux (*radical scavengers*), perd son caractère de radical libre avec une diminution de sa couleur.

En raison de son caractère stable, le DPPH a également été utilisé dans des techniques *EPR* pour mesurer l'activité de désactivation de radicaux libres de l'épiderme humain, comme décrit dans l'article HPLC analysis of vitamin E isoforms in human epidermis: Correlation with minimal erythema dose and free. radical scavenger activity: Fuchs J, Weber, S, Podda M, Groth N, Herrling T, Packer L, Kaufmann R, Free radic. Biol. Med. 2003 Feb. 1; 34 (3) : 330-6.

Les techniques d'analyse actuelles mettant en oeuvre le DPPH restent relativement complexes et coûteuses.

Il est connu d'utiliser des patches afin de détecter la présence d'analytes, autres que les antioxydants, sur la peau.

Le brevet EP 1262559 décrit un patch permettant de détecter la présence de lactate sur la peau par l'intermédiaire d'une première réaction avec une enzyme présente sur le patch conduisant à la formation du pyruvate ou d'un autre sous-produit. Ces produits peuvent être détectés en réagissant avec un autre réactif également présent sur le patch.

Le brevet US 4595011 divulgue un dosimètre transdermique sous forme d'un patch récoltant les substances contenues dans la sueur et faisant réagir cette dernière sur des réactifs présents sur le patch produisant un changement de couleur de patch.

Il existe par conséquent un besoin pour bénéficier d'un moyen permettant d'évaluer facilement le potentiel antioxydant de la peau, dans le but par exemple de déterminer si la peau dispose de ressources suffisantes en substances désactivatrices de radicaux libres et si un apport supplémentaire en substances antioxydantes peut être souhaitable.

L'invention vise notamment à répondre à ce besoin.

L'invention a pour objet un procédé pour évaluer le potentiel de désactivation de radicaux libres de la peau, tel que défini dans les revendications, comportant les étapes consistant à :
- prélever de manière non invasive sur la peau au moins un analyte désactivateur de radicaux libres présent à la surface de celle-ci,
- permettre à l'analyte ainsi prélevé de réagir au contact d'un réactif présent sur un substrat et capable de produire une réaction observable optiquement en présence de l'analyte.

L'invention favorise une concentration relativement élevée en analytes et en réactif du fait que ceux-ci peuvent être présents sur de faibles épaisseurs lors de leur mise en contact, ce qui facilite la détection d'un changement de couleur.

Le prélèvement peut s'effectuer directement en appliquant le substrat sur la zone de la peau comportant l'analyte à prélever.

Ce changement de couleur peut être détecté visuellement et/ou au moyen d'un appareil, par exemple un chromamètre ou autrement encore, notamment à l'aide d'un scanner.

Une échelle colorimétrique est utilisée afin de faciliter la détection visuelle du changement de couleur.

L'invention permet ainsi de bénéficier d'un moyen simple à mettre en oeuvre pour évaluer le potentiel de désactivation de radicaux libres de la peau.

Le prélèvement comporte par exemple la solubilisation d'un ou plusieurs analytes désactivateurs de radicaux libres présents à la surface de la peau avec un liquide de prélèvement dans lequel le réactif est de préférence soluble, par exemple un liquide comportant un alcool, notamment une solution d'éthanol.

Le liquide servant au prélèvement peut imprégner au moins au moment du prélèvement un support poreux, ce qui peut simplifier l'opération de prélèvement et, le cas échéant, le conditionnement de ce liquide. Le support poreux est par exemple un support comportant des fibres, étant par exemple ouaté.

Le support est par exemple monté à l'extrémité d'un tube. Ce dernier peut, le cas échéant, être creux et contenir le liquide de prélèvement.

Le substrat précité, portant le réactif, peut être différent du support poreux.

Le substrat peut notamment être une membrane, celle-ci pouvant être poreuse, avec de préférence une porosité comprise entre 0,4 et 0,5 µm, par exemple de l'ordre de 0,45 µm.

De préférence, le substrat chargé du réactif est sec. Dans une variante, le substrat chargé du réactif est conditionné à l'état humide, le substrat étant par exemple imprégné du liquide de prélèvement.

Le matériau du substrat est par exemple choisi parmi le polyamide, la cellulose, l'acétate de cellulose, le polytétrafluoroéthylène, le polycarbonate, le polyéther sulfone, le fluorure de polyvinilidone.

La concentration du réactif sur le substrat est par exemple de 0,2 mg à 20 mg par 100 cm², de préférence environ 2 mg/100 cm², notamment lorsque le substrat est une membrane poreuse.

Le réactif comporte par exemple du DPPH présentant un caractère de radical libre stable et capable de produire une réaction observable optiquement en présence d'une substance désactivatrice de radicaux libres.

Le substrat est de préférence chargé positivement.

Le réactif peut avoir été déposé sur le substrat par pulvérisation, sérigraphie, jet d'encre ou imprégnation, et avoir subi le cas échéant un séchage plus ou moins prononcé.

Avant d'effectuer le prélèvement, on peut délimiter sur la peau le contour de la surface sur laquelle le prélèvement doit avoir lieu, afin de traiter une surface de peau prédéfinie lors de l'opération de prélèvement et améliorer ainsi la précision de l'évaluation et faciliter la comparaison des résultats.

Le contour de la région sur laquelle le prélèvement est effectué est par exemple défini en appliquant sur la peau un masque, notamment un masque adhésif, ce masque étant par exemple une feuille de papier revêtue sur une face d'un adhésif permettant le repositionnement.

Un tel masque comporte par exemple une fenêtre de dimensions prédéfinies.

Lorsque le liquide de prélèvement est contenu dans un tube, ce dernier peut présenter une extrémité sécable, laquelle est rompue au moment de l'utilisation de manière à permettre l'écoulement du liquide de prélèvement vers le support poreux, présent à l'autre extrémité du tube.

Dans la mise en oeuvre de l'invention, le support poreux est utilisé pour recueillir le ou les analytes à la surface de la peau, notamment en étant amené imprégné du liquide de prélèvement à son contact, puis le support est amené au contact du substrat chargé du réactif.

Egalement décrit est un substrat chargé d'un réactif présentant un caractère de radical libre stable, capable de produire une réaction observable optiquement en présence d'au moins un analyte de la peau désactivateur de radicaux libres.

Le substrat peut comporter une membrane, notamment une membrane poreuse, et le réactif peut être initialement coloré.

Le réactif comporte par exemple du DPPH.

Le substrat peut être un matériau polymère, notamment choisi parmi le polyamide, la cellulose, l'acétate de cellulose, le polytétrafluoroéthylène, le polycarbonate, le polyéther sulfone, le fluorure de polyvinilidone.

Le substrat présente avantageusement une résistance aux solvants tels que l'éthanol. La porosité du substrat peut faciliter l'accrochage des macromolécules biologiques. Le substrat peut comporter une matrice fibreuse d'un non tissé, par exemple des fibres de polyester, qui lui confère une résistance mécanique améliorée. Cette matrice fibreuse peut être noyée dans du NYLON^{®} 6,6, comme dans le cas des membranes BIODYNE de la société PALL.

Le substrat peut être sec.

Le substrat peut être chargé seulement superficiellement en réactif ou, en variante, plus profondément en réactif, par exemple suite à une imprégnation complète avec le réactif

Egalement décrit est un kit permettant d'évaluer le potentiel de désactivation de radicaux libres de la peau, ce kit comportant au moins un substrat tel que défini plus haut.

Le kit comporte avantageusement un élément de prélèvement permettant de prélever de manière non invasive au moins un analyte désactivateur de radicaux libres présent à la surface de la peau, en vue de le faire réagir avec le réactif

L'élément de prélèvement peut être agencé pour permettre le conditionnement d'un liquide servant au prélèvement, avant l'utilisation. L'élément de prélèvement se présente ainsi par exemple sous la forme d'un tube creux rempli du liquide de prélèvement, pourvu d'une extrémité sécable et à l'autre extrémité d'un support poreux dans lequel le liquide de prélèvement peut s'écouler après rupture de l'extrémité sécable.

Le kit peut comporter une échelle colorimétrique ainsi qu'un moyen permettant de délimiter une région de peau sur laquelle le prélèvement doit avoir lieu, par exemple un masque, notamment un masque adhésif.

Egalement décrit est un procédé pour mettre en évidence l'effet d'une action sur le potentiel de désactivation des radicaux libres de la peau, dans lequel :
a) on détermine le potentiel de désactivation des radicaux libres de la peau, par exemple par la mise en oeuvre du procédé tel que défini plus haut,
b) on effectue une action susceptible d'agir sur ce potentiel,
c) on détermine à nouveau ce potentiel après cette action, et l'on évalue l'incidence de celle-ci sur le potentiel par comparaison des résultats avant et après l'action.

Cette dernière comporte par exemple l'application sur la peau d'un produit et/ou l'ingestion d'au moins un complément alimentaire, ou l'exposition à un environnement particulier, par exemple le soleil.

L'effet des ultraviolets sur le potentiel de désactivation de radicaux libres de la peau peut ainsi être mis en évidence, et les résultats peuvent être utilisés par exemple dans une campagne de sensibilisation des personnes aux effets du soleil et/ou pour promouvoir la vente de produits visant à diminuer le stress cutané lié au soleil.

Le kit précité peut comporter en outre, le cas échéant, un produit à appliquer sur la peau, par exemple un produit de protection solaire ou visant à retarder le vieillissement cutané.

Dans un exemple de mise en oeuvre de l'invention, le potentiel de désactivation de radicaux libres de la peau est évalué avant et après exposition à une source de stress cutané, par exemple le soleil, dans un cas où la peau n'est pas protégée et dans un autre cas où la peau est protégée, et les résultats sont comparés afin de mettre en évidence l'effet protecteur du produit appliqué sur la peau.

Egalement décrit est un procédé dans lequel on évalue le potentiel de désactivation de radicaux libres de la peau en mettant en oeuvre un procédé d'évaluation tel que défini ci-dessus et dans lequel on délivre ensuite un conseil relatif à un produit et/ou à un traitement destiné à avoir un effet sur ce potentiel, en fonction du résultat de l'évaluation.

Egalement décrit est un procédé de formulation d'une composition cosmétique ou dermatologique personnalisée, dans lequel on évalue le potentiel de désactivation de radicaux libres de la peau d'une personne et l'on détermine pour cette personne la teneur de la composition en au moins une substance antioxydante en fonction du potentiel ainsi déterminé. Cet antioxydant est par exemple choisi parmi : la vitamine E, (alpha tocophérols et isomères), acide ascorbique, coenzyme Q, acide urique, caroténoïdes, flavonoïdes et polyphénols, aminoindoles et mélatonine, acide dihydrolipoique, etc ...

L'invention pourra être mieux comprise à la lecture de la description détaillée qui va suivre, d'exemples de mise en oeuvre de celle-ci, et à l'examen du dessin annexé, sur lequel :
- la figure 1 représente de manière schématique, en perspective, un exemple de kit,
- les figures 2 et 3 illustrent l'utilisation du kit,
- la figure 4 illustre l'utilisation d'un appareil pour détecter un changement de couleur,
- la figure 5 illustre le traitement à distance de données résultant de l'évaluation du potentiel de désactivation de radicaux libres de la peau,
- la figure 6 représente un ensemble comportant le kit de la figure 1 et un produit associé, et
- les figures 7 et 8 représentent deux variantes de réalisation.

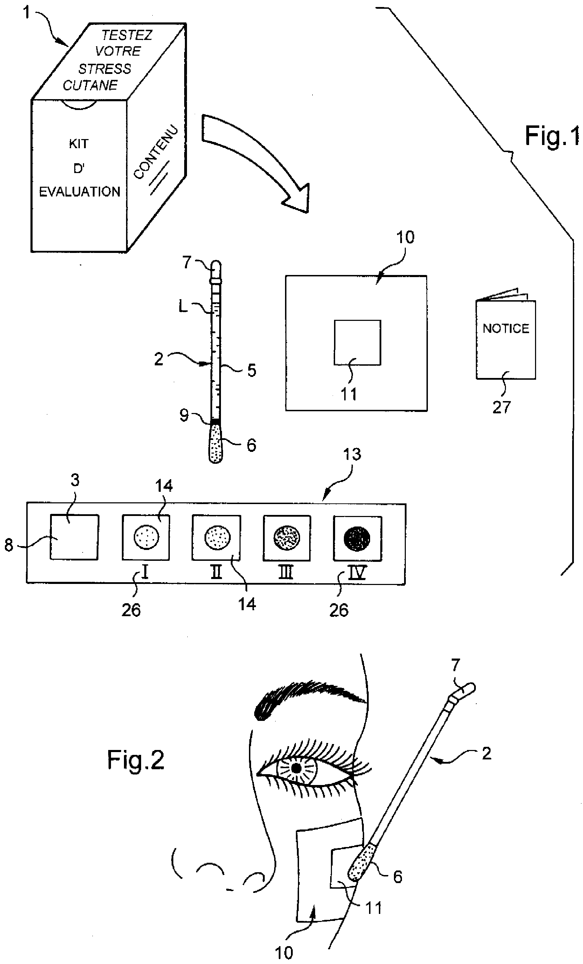

Le kit 1 représenté à la figure 1 comporte un élément de prélèvement 2 destiné à prélever un ou plusieurs analytes à la surface de la peau et un réactif 3 permettant de produire en présence de ce ou ces analytes une réaction observable optiquement.

L'élément de prélèvement 2 comporte par exemple un tube 5 rempli d'un liquide de prélèvement L, ce tube 5 étant muni à une extrémité d'un support poreux 6 et à l'autre extrémité d'une partie sécable 7. Lorsque cette dernière est rompue, cela crée une prise d'air dans le tube 5 au-dessus du liquide L et ce dernier peut s'écouler dans le support 6.

Des exemples d'éléments de prélèvement pouvant convenir sont décrits dans la demande de brevet US 2004/0158188 et peuvent être fabriqués par la société américaine SWAB PLUS.

L'élément de prélèvement 2 peut comporter dans le tube 5 au-dessus du support 6 un bouchon 9 d'un liquide non miscible avec le liquide de prélèvement, de manière à isoler le liquide de prélèvement L de l'extérieur. Ce bouchon 9 est par exemple un ménisque d'une huile de silicone, capable de s'évacuer avec le liquide de prélèvement L lorsque ce dernier quitte le tube 5.

Le support 6 est par exemple un embout ouaté.

Le liquide de prélèvement L est par exemple une solution d'éthanol à 10 %.

Le réactif 3 est par exemple du DPPH déposé sur un substrat 8 choisi de manière à ne pas affecter sa réactivité et de préférence poreux de façon à faciliter son imprégnation par le réactif

Dans l'exemple considéré, le substrat 8 est une membrane résistant aux solvants organiques tels que l'éthanol et ne contenant pas de trace de peroxydes susceptible de diminuer la sensibilité du réactif aux substances antioxydantes et ne contenant pas de substances antioxydantes qui pourraient réduire le réactif et affecter la détection.

Le substrat 8 est par exemple une membrane en polyamide, par exemple une membrane en NYLON^{®} 6,6 chargée positivement. La membrane présente une surface poreuse avec une taille de pores par exemple de 0,2, 0,45 ou 1,2 µm, de préférence de 0,45 µm.

L'épaisseur de la membrane est par exemple de 6.0 mils plus ou moins 0,5 mils.

Des membranes pouvant convenir sont commercialisées par la société américaine PALL CORPORATION sous les références commerciales BIODYNE B ou C.

La membrane 8 peut se présenter par exemple sous la forme d'une pastille indépendante ou être, comme dans l'exemple illustré, disposée à côté de témoins colorés 14 au sein d'une échelle colorimétrique 13 permettant d'évaluer plus facilement un changement de couleur de celle-ci.

Les témoins colorés 14 sont par exemple associés à des identifiants 26 alphanumériques ou autres, permettant de les distinguer.

Le kit 1 peut comporter une notice 27 renseignant l'utilisateur du kit 1 sur la manière de conduire le test et sur l'analyse qu'il convient de faire des résultats de l'évaluation, en fonction par exemple de l'identifiant 26 correspondant au changement de coloration observé.

Dans un exemple de mise en oeuvre de l'invention, la membrane 8 se présente sous la forme d'une pastille carrée de 2 cm de côté, réalisée à partir d'une feuille de 10 cm de côté de BIODYNE B ou C, préalablement chargée du réactif 3 par dépôt de 2 ml d'une solution de DPPH de la société allemande SIGMA ALDRICH CHEMIE, STEINHEIN, à une concentration de 100 mg/100 ml d'éthanol à 96°, puis agitation et séchage sous hotte à température ordinaire pendant deux heures.

La concentration en DPPH sur la membrane est par exemple comprise entre 0,2 mg et 20 mg par 100 cm², étant préférentiellement de l'ordre de 2 mg/100 cm².

Le kit 1 comporte en outre dans l'exemple considéré un masque 10, par exemple en papier du genre POST IT^{®}, dont une face est revêtue d'un adhésif sensible à la pression. Ce masque 10 est traversé par une fenêtre 11.

Lors de l'utilisation, le masque 10 est appliqué sur la région sur laquelle doit être effectué le prélèvement, par exemple le visage dans l'exemple de la figure 2.

L'extrémité sécable 7 est rompue et le liquide de prélèvement L s'écoule dans le support 6. Ce dernier est amené au contact de la région de peau délimitée par la fenêtre 11 et les analytes présents à la surface de celle-ci sont prélevés, en faisant effectuer par exemple au support 6 un balayage horizontal puis vertical de la région de prélèvement.

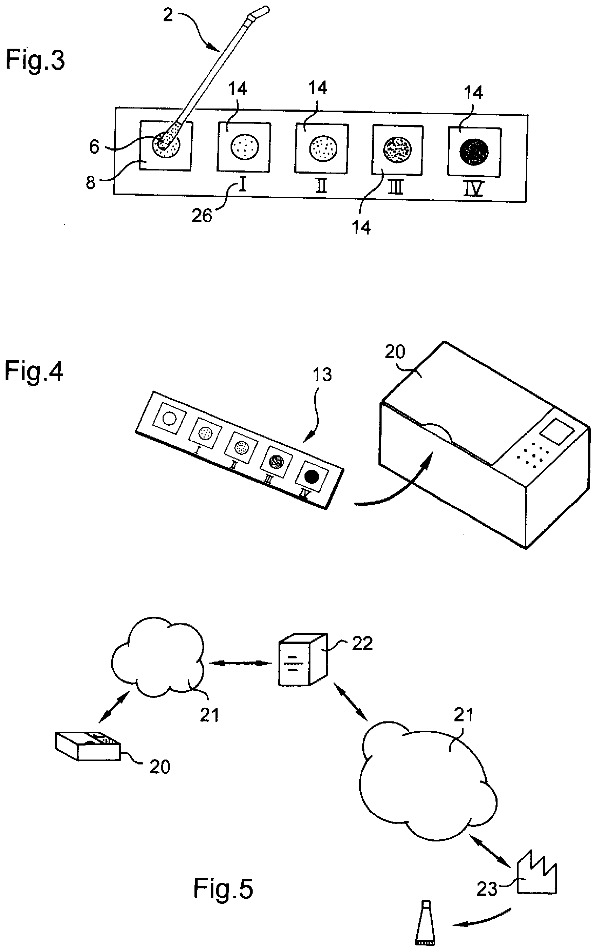

Ensuite, le support 6 est amené au contact par exemple pendant 10 secondes du substrat 8 chargé du réactif 3, comme illustré à la figure 3.

En présence d'une solution à 10 % d'éthanol, le substrat 8 est par exemple rouge/rosé. En présence d'un antioxydant lipophile tel que par exemple la vitamine E, une décoloration est observée, celle-ci étant proportionnelle à la quantité de vitamine E. La décoloration observée peut être évaluée par comparaison avec les témoins colorés 14, après une attente prédéfinie, par exemple de 60 à 120 secondes.

L'évaluation du potentiel de désactivation de radicaux libres de la peau peut être utile par exemple pour mettre en évidence le besoin d'un traitement ou l'effet d'un traitement sur la peau.

Le kit 1 est par exemple utilisé sur un point de vente ou dans un institut de beauté ou chez un dermatologue pour conseiller un consommateur ou un patient sur le besoin éventuel d'un traitement. Dans ce cas, il peut être proposé, au vu du résultat de l'évaluation, un ou plusieurs produits à appliquer sur la peau ou à ingérer.

Le ou les produits à ingérer peuvent par exemple comporter plus de 50 mg de vitamine C, plus de 5 mg de vitamine E, plus de 100 mg de polyphénols de raisin ou de thé, plus de 1 µg de sélénium et/ou plus de 1mg de bétacarotène.

On peut par exemple proposer une formule orale correspondant à une dose journalière de 60 mg de vitamine C, 10 mg de vitamine E, 200 mg de polyphénols de raisin ou de thé, 2 µg de sélénium et 2 mg de bétacarotène.

Le kit peut encore être utilisé au domicile du consommateur. Dans ce cas, le consommateur peut procéder lui-même au prélèvement puis à la détection d'un changement de couleur du réactif.

Que l'évaluation soit effectuée à domicile ou non, un appareil peut être utilisé pour faciliter la détection d'un changement de couleur du réactif.

Une mesure de l'absorption à une longueur d'onde prédéfinie, par exemple 517 nm, peut par exemple permettre de détecter une variation de la couleur du réactif. Un chromamètre peut encore être utilisé.

Dans un exemple de mise en oeuvre de l'invention, la comparaison de la couleur du substrat 8 avec celle des témoins colorés 14 est effectuée avec l'aide d'un scanner couleur 20, ce scanner étant par exemple intégré à une imprimante couleur multifonction, comme illustré à la figure 4.

Cette imprimante peut être connectée à un micro-ordinateur ou terminal mobile non représenté, chargé d'analyser l'image ainsi numérisée afin de détecter le changement de couleur.

Le scanner peut encore être relié, directement ou non, à un réseau informatique 21, par exemple Internet, et avoir téléchargé par ce réseau 21 un programme permettant d'effectuer cette comparaison, comme illustré à la figure 5.

Le cas échéant, le traitement des données peut être effectué par un serveur distant 22, auquel par exemple l'image numérisée est transmise.

Ce serveur 22 peut ensuite transmettre des informations au consommateur, notamment un résultat d'analyse, lequel peut être accompagné par la prescription d'un produit, voire par la fourniture d'un produit personnalisé, le serveur 22 ayant par exemple communiqué, par exemple par l'intermédiaire du réseau 2, des informations à un centre de fabrication 23.

Le produit personnalisé comporte par exemple une substance antioxydante dont la concentration est fonction du potentiel de désactivation de radicaux libres de la peau précédemment évalué.

Cette substance est par exemple choisie parmi la vitamine E, la vitamine C, le NDGA, les thiols et ses dérivés (Glutathion, N-acétylcystéine), les oligomères procyanidoliques (OPC), les flavonoides, les catéchines, épi catéchines ainsi que ses dérivés galliques, les polyphénols comme par exemple le tyrosol, l'hydroxytyrosol, le sésamol, le carnosol, le gamma orizanol, les acides tels que férulique, caféique, rosemarinique, carnosique, etc ...

L'invention peut encore être mise en oeuvre pour évaluer l'effet d'un traitement sur la peau.

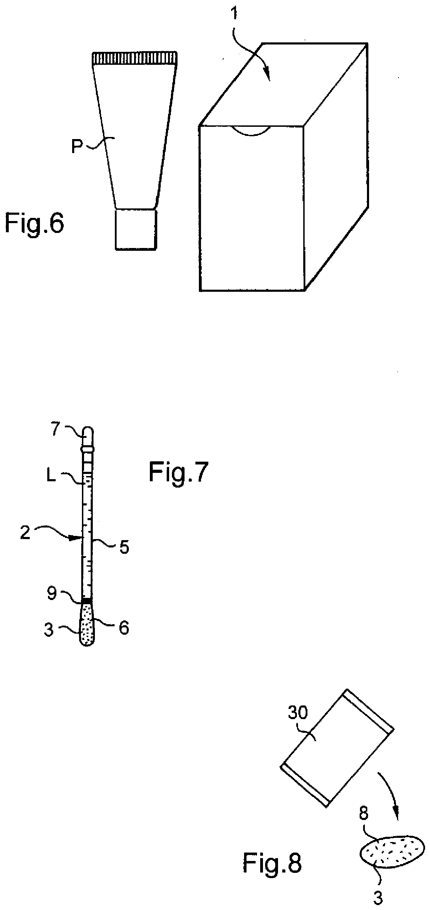

Le kit 1 est par exemple proposé en accompagnement d'un produit P à appliquer sur la peau, étant par exemple commercialisé avec ce produit, comme illustré sur la figure 6.

L'utilisateur peut par exemple évaluer le potentiel de désactivation des radicaux libres de la peau avant l'utilisation du produit puis après celle-ci et en comparant les résultats déterminer l'effet du traitement sur ce potentiel.

Le kit d'évaluation peut encore être utilisé afin de déterminer une posologie d'un produit ayant un effet sur le potentiel antioxydant de la peau, par exemple un nombre d'applications du produit par intervalle de temps ou une quantité à appliquer.

Le kit d'évaluation 1 peut encore être utilisé pour déterminer des régions du corps à traiter, en faisant par exemple apparaître que certaines régions du corps ont un potentiel de désactivation de radicaux libres plus élevé que d'autres, ces dernières ayant par conséquent un besoin plus grand de traitement.

L'invention peut encore être utile pour mettre en évidence l'effet d'un environnement sur la peau, par exemple pour évaluer le stress cutané induit par l'exposition au soleil.

Deux évaluations de régions du corps, respectivement exposée et non exposée au soleil, peuvent être effectuées et les résultats comparés. Une telle comparaison peut mettre en évidence le stress cutané plus ou moins important selon les individus engendré par le soleil.

Un identifiant déterminé au cours de l'évaluation du potentiel de la désactivation des radicaux libres de la peau ou de l'évaluation de la résistance de la peau à un stress cutané, peut figurer sur des produits ou dans des tables de correspondance associées à des produits, afin de permettre à l'utilisateur connaissant le potentiel antioxydant de sa peau ou la résistance de celle-ci à un stress générateur de radicaux libres, de choisir des produits adaptés.

L'utilisation d'une membrane pour véhiculer le réactif 3 ne constitue qu'un exemple parmi d'autres de substrats.

Par exemple, le support 6 de l'élément de prélèvement 2 peut être utilisé également comme substrat pour le réactif, comme illustré figure 7.

L'élément de prélèvement 2 peut alors être utilisé de la manière suivante.

L'extrémité sécable 7 est rompue, ce qui permet au liquide de prélèvement d'imbiber le support 6, puis celui-ci est amené au contact de la peau pour recueillir des analytes susceptibles de réagir avec le réactif 3.

Un changement de couleur éventuel est alors observé directement sur le support 6.

Ce dernier peut être différent d'un embout ouaté le cas échéant, de manière à faciliter la détection visuelle du changement de couleur.

Le réactif 3 peut encore être présent, comme illustré figure 8, sur une lingette, pastille ou patch, un tel substrat étant préimprégné par le liquide de prélèvement et conditionné de manière hermétique dans un sachet 30.

Pour utiliser un tel élément de prélèvement, l'utilisateur sort le substrat, qui est également le support du liquide de prélèvement, du sachet 30, puis applique le substrat sur la peau de manière à recueillir les analytes qui se trouvent à sa surface.

La réaction colorée est observée sur le substrat.

Dans toute la description, y compris les revendications, l'expression « comportant un » doit être comprise comme étant synonyme de « comportant au moins un », sauf si le contraire est spécifié.

## Revendications

1. Procédé pour évaluer le potentiel antioxydant de la peau, comportant les étapes consistant à :
- prélever de manière non invasive sur la peau au moins un analyte désactivateur de radicaux libres présent à la surface de celle-ci, en utilisant un support poreux (6), pour recueillir le ou les analyte(s) à la surface de la peau, ledit support poreux étant imprégné d'un liquide de prélèvement (L), le prélèvement comportant la solubilisation du ou des analyte(s) avec le liquide de prélèvement (L),
- puis amener ledit support poreux au contact d'un substrat (8) différent du support poreux, ledit substrat étant chargé d'un réactif (3) comportant du DPPH, et
- permettre à l'analyte ainsi prélevé de réagir au contact du réactif (3) présent sur ledit substrat (8), ledit substrat étant capable de produire une réaction observable optiquement en présence de l'analyte,
**caractérisé par le fait qu'**une échelle colorimétrique (13) est utilisée lors de la détection visuelle d'un changement de couleur du réactif.

2. Procédé selon la revendication 1, **caractérisé par le fait que** le réactif (3) est soluble dans le liquide de prélèvement.

3. Procédé selon la revendication 1 ou 2, **caractérisé par le fait que** le liquide de prélèvement comporte un alcool, notamment de l'éthanol.

4. Procédé selon la revendication 3, **caractérisé par le fait que** le support poreux (6) comporte des fibres.

5. Procédé selon les revendications 1 à 4, **caractérisé par le fait que** le support poreux (6) est disposé à l'extrémité d'un tube.

6. Procédé selon la revendication 5, **caractérisé par le fait que** le support (6) est ouaté.

7. Procédé selon la revendication 5 ou 6, **caractérisé par le fait qu'**un tube (5) contient le liquide de prélèvement.

8. Procédé selon l'une quelconque des revendications précédentes, **caractérisé par le fait que** le substrat (8) est une membrane.

9. Procédé selon l'une quelconque des revendications précédentes, **caractérisé par le fait que** le substrat (8) est poreux, étant notamment réalisé dans un matériau choisi parmi le polyamide, la cellulose, l'acétate de cellulose, le polytétrafluoroéthylène, le polycarbonate, le polyéther sulfone, le fluorure de polyvinilidone.

10. Procédé selon l'une quelconque des revendications 1 à 10, **caractérisé par le fait qu'**avant d'effectuer le prélèvement, le contour de la surface sur laquelle le prélèvement doit avoir lieu est délimité sur la peau.

11. Procédé selon la revendication précédente, **caractérisé par le fait que** ledit contour est délimité en appliquant sur la peau un masque (10), notamment un masque adhésif.

12. Procédé selon la revendication 7, **caractérisé par le fait que** le tube (5) présente une extrémité sécable (7), laquelle est rompue au moment de l'utilisation, de manière à permettre l'écoulement du liquide de prélèvement vers le support poreux.

## Patentansprüche

1. Verfahren zum Bewerten des Antioxidationspotentials der Haut mit den Schritten, die darin bestehen:
- eine Probe von mindestens einem Deaktivierungsanalyten von freien Radikalen an der Haut, der an ihrer Oberfläche vorhanden ist, in nicht invasiver Weise unter Verwendung eines porösen Trägers (6) zu nehmen, um den oder die Analyten an der Oberfläche der Haut zu sammeln, wobei der poröse Träger mit einer Probennahmeflüssigkeit (L) imprägniert ist, wobei die Probennahme die Solubilisierung des oder der Analyten mit der Probennahmeflüssigkeit (L) umfasst,
- dann den porösen Träger in Kontakt mit einem Substrat (8) zu bringen, das vom porösen Träger verschieden ist, wobei das Substrat mit einem Reagens (3) mit DPPH beladen ist, und
- zu ermöglichen, dass der so entnommene Analyt in Kontakt mit dem Reagens (3), das an dem Substrat (8) vorhanden ist, reagiert, wobei das Substrat eine Reaktion erzeugen kann, die in Gegenwart des Analyten optisch beobachtbar ist,
- **dadurch gekennzeichnet, dass** eine Farbskala (13) bei der visuellen Detektion einer Farbänderung des Reagens verwendet wird.

2. Verfahren nach Anspruch 1, **dadurch gekennzeichnet, dass** das Reagens (3) in der Probennahmeflüssigkeit löslich ist.

3. Verfahren nach Anspruch 1 oder 2, **dadurch gekennzeichnet, dass** die Probennahmeflüssigkeit einen Alkohol, insbesondere Ethanol, umfasst.

4. Verfahren nach Anspruch 3, **dadurch gekennzeichnet, dass** der poröse Träger (6) Fasern umfasst.

5. Verfahren nach den Ansprüchen 1 bis 4, **dadurch gekennzeichnet, dass** der poröse Träger (6) am Ende eines Rohrs angeordnet ist.

6. Verfahren nach Anspruch 5, **dadurch gekennzeichnet, dass** der Träger (6) wattiert ist.

7. Verfahren nach Anspruch 5 oder 6, **dadurch gekennzeichnet, dass** ein Rohr (5) die Probennahmeflüssigkeit enthält.

8. Verfahren nach einem der vorangehenden Ansprüche, **dadurch gekennzeichnet, dass** das Substrat (8) eine Membran ist.

9. Verfahren nach einem der vorangehenden Ansprüche, **dadurch gekennzeichnet, dass** das Substrat (8) porös ist, wobei es insbesondere aus einem Material hergestellt ist, das aus Polyamid, Cellulose, Celluloseacetat, Polytetrafluorethylen, Polycarbonat, Polyethersulfon, Polyvinylidenfluorid ausgewählt ist.

10. Verfahren nach einem der Ansprüche 1 bis 10, **dadurch gekennzeichnet, dass** vor dem Durchführen der Entnahme die Kontur der Oberfläche, an der die Entnahme stattfinden soll, auf der Haut begrenzt wird.

11. Verfahren nach dem vorangehenden Anspruch, **dadurch gekennzeichnet, dass** die Kontur durch Aufbringen einer Maske (10), insbesondere einer klebenden Maske, auf die Haut begrenzt wird.

12. Verfahren nach Anspruch 7, **dadurch gekennzeichnet, dass** das Rohr (5) ein teilbares Ende (7) aufweist, das im Moment der Verwendung abgebrochen wird, um das Fließen der Probennahmeflüssigkeit zum porösen Träger zu ermöglichen.

## Claims

1. A method for evaluating the anti-oxidative potential of the skin, including the steps consisting of:
- non-invasively sampling on the skin at least one analyte deactivating free radicals present at the surface thereof, by using a porous support (6), for collecting the analyte(s) at the surface of the skin, said porous support being impregnated with a sampling liquid (L), the sample including solubilization of the analyte(s) with the sampling liquid (L),
- and of then bringing said porous support into contact with a substrate (8) different from the porous support, said substrate being loaded with a reagent (3) including DPPH, and
- allowing the thereby sampled analyte to react upon contact with the reagent (3) present on said substrate (8), said substrate being capable of producing an optically observable reaction in the presence of the analyte,
**characterized by** the fact that a colorimetric scale (13) is used upon visual detection of a color change of the reagent.

2. The method according to claim 1, **characterized by** the fact that the reagent (3) is soluble in the sampled liquid.

3. The method according to claim 1 or 2, **characterized by** the fact that the sampled liquid includes an alcohol, notably ethanol.

4. The method according to claim 3, **characterized by** the fact that the porous support (6) includes fibers.

5. The method according to claims 1 to 4, **characterized by** the fact that the porous support (6) is positioned at the end of a tube.

6. The method according to claim 5, **characterized by** the fact that the support (6) is fleecy.

7. The method according to claim 5 or 6, **characterized by** the fact that a tube (5) contains the sampled liquid.

8. The method according to any of the preceding claims, **characterized by** the fact that the substrate (8) is a membrane.

9. The method according to any of the preceding claims, **characterized by** the fact that the substrate (8) is porous, being notably made in a material selected from among polyamide, cellulose, cellulose acetate, polytetrafluoroethylene, polycarbonate, polyether sulfone, polyvinylidene fluoride.

10. The method according to any of claims 1 to 10, **characterized by** the fact that before carrying out the sampling, the contour of the surface on which the sampling should take place is delimited on the skin.

11. The method according to the preceding claim, **characterized by** the fact that said contour is delimited by applying a mask (10), notably an adhesive mask.

12. The method according to claim 7, **characterized by** the fact that the tube (5) has a divisible end (7), which is broken upon use, so as to allow flow of the sampled liquid towards the porous support.
